# FASCICULE DE BREVET EUROPEEN

(11) **EP 0 793 947 B1**
(45) Date de publication et mention de la délivrance du brevet: **19.06.2002**
(21) Numéro de dépôt: 97450002.7
(22) Date de dépôt: 04.03.1997
(51) Int. Cl.: A61B 17/70

(54) **Dispositif de liaison rachidienne transverse**
Transversale Wirbelsäulenverbindungsvorrichtung
Transverse spinal connection device

(30) Priorité: 05.03.1996 FR 9603053
(43) Date de publication de la demande: 10.09.1997
(73) Titulaire: Brienne Industries S.à.r.l., 33600 Pessac (FR); Franck, Bruno, 87180 Isle (FR); Alphamed S.à.r.l., 64210 Bidart (FR)
(72) Inventeur: Martin, Alain, 33160 Saint Medard en Jalles (FR); Franck, Bruno, 87180 Isle (FR)
(74) Mandataire: Thébault, Jean-Louis

(56) Documents cités:
- EP-A- 0 446 092
- DE-A- 3 924 050

## Description

La présente invention a trait à un dispositif de liaison rachidienne transverse utilisable en neurochirurgie, en chirurgie orthopédique et plus globalement en chirurgie du rachis et destiné à constituer une liaison d'entretoisement en particulier entre deux tiges d'ostéosynthèse rachidienne.

Ces tiges, qui sont des tiges longitudinales de section circulaire, sont destinées à s'adapter aux courbures physiologiques du rachis ou à rétablir ces courbures et doivent la plupart du temps, après mise en place à l'aide de vis pédiculaires ou de tout autre système de fixation postérieure, et afin de permettre une bonne réduction et une bonne contention, être reliées entre elles en un ou plusieurs endroits à l'aide de barres transversales.

Les dispositifs du type à barre solidarisée au croisement avec les tiges de contention à l'aide d'organes de liaison et de blocage présentent des inconvénients. Certains utilisent comme organes de liaison/blocage des sortes de colliers qu'il faut au préalable enfiler sur les tiges de contention avant la solidarisation de ces dernières avec les vis pédiculaires ou analogues.

Ceci ne facilite pas la mise en place des tiges et, de plus, une fois la fixation des tiges effectuée, la ou les barres transversales ne peuvent être changées de place car les colliers de liaison/blocage sont prisonniers des tiges. D'autres dispositifs du même type comportent des organes de liaison/blocage non plus enfilés sur les tiges de contention mais rapportés sur ces dernières, une fois en place, ces organes étant constitués de deux parties venant entourer la tige. Un dispositif de ce type est décrit dans DE-A-3924050.

Si ce système est plus pratique et souple que le précédent, car il permet la mise en place d'une barre de liaison transversale en divers endroits des tiges de contention après leur fixation et un changement de place éventuel, il n'est pas cependant complètement satisfaisant.

En effet, bien que les deux parties desdits organes de liaison/blocage puissent être rapportés sur les tiges de contention in situ, ils entourent cependant complètement les tiges et, de ce fait, nécessitent une intervention locale sur les vertèbres pour dégager l'espace nécessaire pour recevoir lesdites parties, ce qui limite par ailleurs les possibilités d'implantation des barres transversales.

La présente invention a précisément pour but de palier ces inconvénients en proposant un système de liaison transverse adaptable directement notamment sur des tiges d'ostéosynthèse rachidienne in situ, sans intervention préalable pour dégager la place nécessaire et permettant la mise en place des barres transversales véritablement en n'importe quel endroit des tiges de contention, en dehors des vis pédiculaires bien entendu.

A cet effet, l'invention a pour objet un dispositif de liaison rachidienne transverse, du type comprenant une barre transversale solidarisable d'au moins une tige d'ostéosynthèse rachidienne à l'aide d'un organe de liaison/blocage entre ladite barre transversale et ladite tige d'ostéosynthèse, susceptible d'être rapporté sur cette dernière in situ, caractérisé en ce que ledit organe de liaison/blocage est constitué :
- d'une première pièce en forme de mâchoire, munie d'un passage pour la barre transversale et d'une vis de blocage ou analogue susceptible de faire saillie dans ledit passage,
- et d'une seconde pièce en forme de mâchoire antagoniste articulée sur la première pièce,
- la barre en place dans le passage étant susceptible, lors du serrage de ladite vis, de venir en appui à la fois contre la première pièce et contre la seconde pièce et de faire se rapprocher lesdites mâchoires.

Suivant un mode de réalisation préféré, les mâchoires sont configurées de façon à ne pas entourer complètement la tige d'ostéosynthèse, le passage ménagé dans la première pièce s'étend sensiblement orthogonalement à l'axe de la tige d'ostéosynthèse en position entre les mâchoires, la vis est disposée de l'autre côté des mâchoires par rapport à la barre transversale et la seconde pièce est articulée sur la première par une fourche dans laquelle passe ladite barre transversale.

Les forme et dimensions du passage de la barre transversale dans la première pièce sont déterminées de façon à autoriser à ladite barre un débattement multidirectionnel afin, d'une part, de permettre aux mâchoires de s'écarter suffisamment pour venir chevaucher et enserrer la tige d'ostéosynthèse et, d'autre part, de donner éventuellement à la barre transversale une certaine angulation par rapport à la tige d'ostéosynthèse pour tenir compte des conditions d'implantation.

Un tel dispositif se met en place quasi instantanément par exemple pour entretoiser deux tiges d'ostéosynthèse en place, par simple engagement du dispositif par dessus chacune des tiges des mâchoires, en position ouverte, aux endroits désirés, puis, une fois les mâchoires en position, vissage de la vis qui fait pression sur la barre transversale et sur la seconde pièce/mâchoire, laquelle se rapproche de la première en serrant la tige d'ostéosynthèse. En fin de vissage, non seulement la tige d'ostéosynthèse mais également la barre transversale sont bloquées fermement par rapport au dispositif.

On obtient ainsi un blocage mutuel ferme et sûr entre tige d'ostéosynthèse et barre transversale.

Le déblocage du dispositif par exemple pour un éventuel déplacement le long des tiges d'ostéosynthèse est tout aussi facile et rapide.

La manipulation de ce dispositif s'effectue essentiellement de haut en bas, le patient étant allongé, sans avoir à réaliser de geste chirurgical ni latéralement par rapport à la tige (avivement des articulaires), ni médialement, ni encore par dessus la tige d'ostéosynthèse.

En effet, le profil d'extrémité effilé des mâchoires et le fait que ces dernières laissent libre la périphérie de la tige d'ostéosynthèse tournée vers la colonne vertébrale, par exemple sur une étendue de l'ordre d'un tiers de ladite périphérie, permet la mise en place desdites mâchoires sans problème, c'est-à-dire sans avoir à dégager un espace à cet effet à l'aide d'un instrument.

D'autres caractéristiques et avantages ressortiront de la description qui va suivre d'un mode de réalisation du dispositif de l'invention, description donnée à titre d'exemple uniquement et en regard des dessins annexés sur lesquels :
- Figure 1 est une vue schématique d'une ostéosynthèse rachidienne munie d'un dispositif de liaison transverse conforme à l'invention ;
- Figure 2 est une vue en perspective d'un mode de réalisation préféré du dispositif de l'invention ;
- Figures 3a à 3c représentent respectivement une vue en élévation latérale, une vue de droite et une vue de dessus d'une première pièce du dispositif ;
- Figures 4a à 4c représentent respectivement une vue en élévation latérale, une vue de droite et une vue de dessus d'une seconde pièce du dispositif, et
- Figure 5 est une vue en coupe dans l'axe de la barre transversale du dispositif formé des pièces des figures 3a à 3c et 4a à 4c, montées et en position d'utilisation.

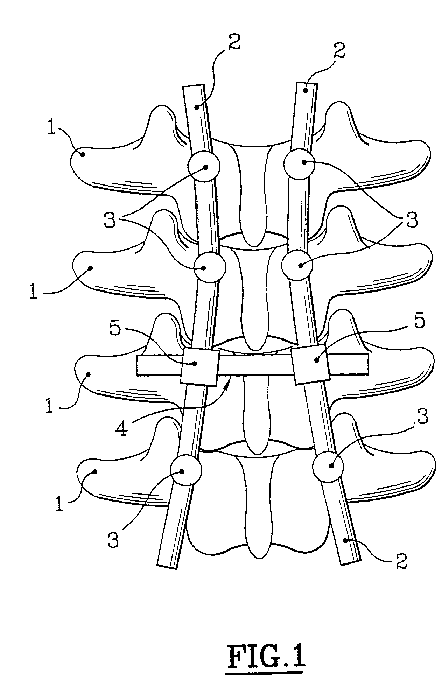

Sur la figure 1, on a représenté schématiquement des vertèbres lombaires 1 sur lesquelles deux tiges 2 d'ostéosynthèse rachidienne sont implantées, à la manière connue à l'aide de vis pédiculaires symbolisées en 3.

Les deux tiges 2 sont entretoisées par un dispositif conforme à l'invention comprenant une barre transversale 4 solidarisée des tiges 2 aux points de croisement par des organes de liaison/blocage schématisés en 5.

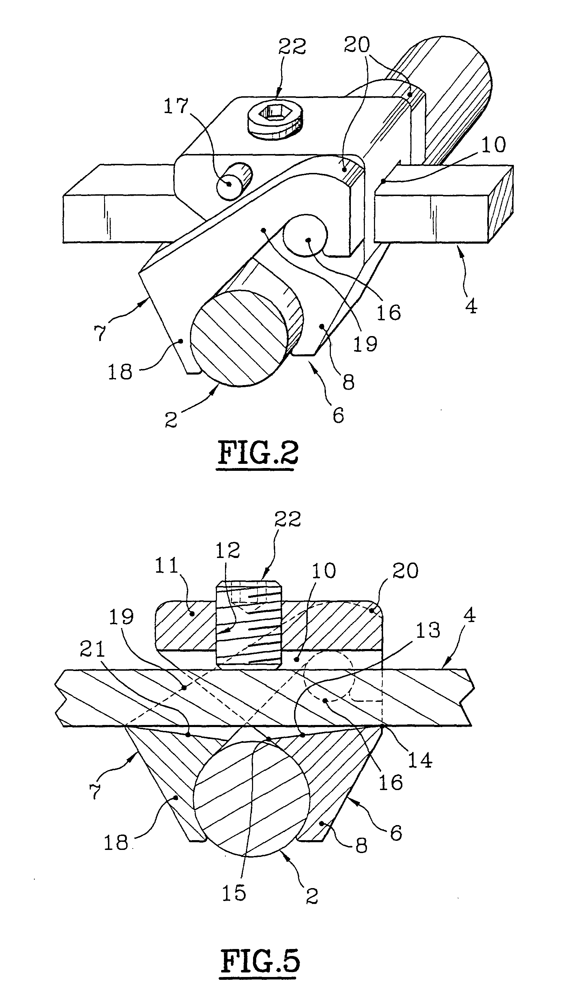

La figure 2 illustre un mode de réalisation préféré du dispositif de l'invention, lequel est constitué, d'une part, d'une barre de liaison transversale 4 de section rectangulaire et, d'autre part, d'un organe de liaison/blocage 5 entre la barre 4 et une tige cylindrique 2 d'ostéosynthèse rachidienne comprenant une première pièce 6 formant une première mâchoire et une seconde pièce 7 articulée sur la pièce et formant une mâchoire antagoniste, les deux mâchoires enserrant la tige d'ostéosynthèse 2.

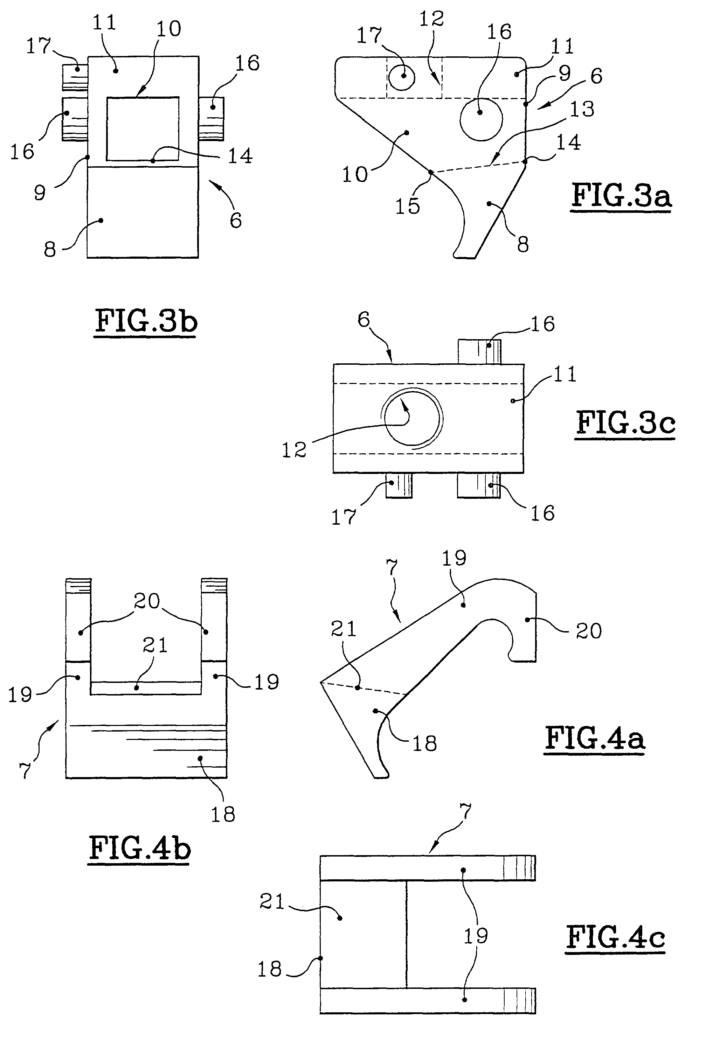

Les figures 3a à 3c et 4a à 4c représentent des pièces 6 et 7 selon la figure 2.

La première pièce 6 représentée par les figures 3a à 3c comporte une extrémité effilée formant mâchoire 8 à face active cylindrique. La mâchoire 8 se prolonge par une partie centrale 9 traversée par un passage 10 de section rectangulaire d'axe sensiblement orthogonal à l'axe du cylindre définissant la face active de la mâchoire 8.

Enfin, la partie centrale 9 est elle-même prolongée par une extrémité opposée à la mâchoire 8 et en forme de table rectangulaire 11 dans laquelle est percé un trou taraudé 12 débouchant dans ledit passage 10.

Le passage 10 présente une section d'entrée qui va, pour ce qui concerne la hauteur et en considérant la figure 3a, en s'agrandissant de la droite vers la gauche en sorte que le plancher 13 dudit passage 10 présente un point haut (arête 14) à l'entrée et un point bas 15 à son autre extrémité.

Sur les flancs opposés de la pièce 6, à hauteur de la partie centrale 9 sont prévus deux tourillons 16 sur lesquels s'articule la pièce 7.

Enfin, sur l'un desdits flancs est également prévue à hauteur de la table 11 une goupille amovible 17 de maintien de la pièce 7 montée sur la pièce 6, comme on le verra plus loin.

La seconde pièce 7 représentée sur les figures 4a à 4c est conformée en mâchoire 18 antagoniste de la mâchoire 8, les deux mâchoires étant sensiblement symétriques.

La mâchoire 18 se prolonge par une fourche à deux branches 19 dont les extrémités sont recourbées en 20 de façon à s'articuler sur les tourillons 16 de la pièce 6. Entre les branches 19 s'étend une face 21 de la mâchoire 18 qui correspond au plancher 13 de la pièce 6. Les pièces 6, 7 sont montées et s'articulent comme illustré par la figure 2, la goupille 17 étant insérée dans la pièce 6 après mise en place de la pièce 7 sur la pièce 6. L'une des branches 19 de la pièce 7 est ainsi retenue prisonnière entre la goupille 17 et l'un des tourillons 16, la pièce 7 ayant un certain débattement angulaire pour ouvrir la paire de mâchoires 8-18 suffisamment pour le passage entre elles de la tige 2 à bloquer.

La figure 5 représente en coupe les pièces 6 et 7 des figures 3a à 3c et 4a à 4c, assemblées et bloquant mutuellement la tige 2 et la barre 4.

Le mode d'emploi du dispositif est le suivant :

Les deux pièces 6, 7 étant assemblées et verrouillés par la goupille 17, la barre 4 est introduite dans le passage 10 et entre les branches 19 de la fourche, une vis 22, par exemple sans tête et à empreinte hexagonale en creux, étant insérée dans le trou taraudé 12 sans faire saillie dans le passage 10 pour ne pas gêner l'engagement de la barre transversale 4.

A l'endroit de la tige 2 en place où l'on désire bloquer la barre 4, on présente les mâchoires 8, 18 contre la tige 2. La pression exercée fait s'ouvrir les mâchoires qui viennent alors entourer la tige 2. Il suffit ensuite de visser la vis 22. Cette dernière fait saillie dans le passage 10, prend appui contre la face en regard de la barre 4 qui elle-même bascule autour d'un axe parallèle à l'axe d'articulation (tourillons 16) des pièces 6, 7, en prenant appui sur l'arête d'entrée 14.

En basculant la barre 4 appuie sur la pièce 7 entre les branches 19 et la fait pivoter en sorte de faire rapprocher la mâchoire 18 de la mâchoire 8, coinçant ainsi la tige 2.

En fin de vissage, la tige 2 est fermement bloquée entre les mâchoires 8, 18 et la barre 4 est fermement bloquée entre la vis 22 et la pièce 7.

La hauteur du passage 10 est notablement supérieure à l'épaisseur de la barre 4 pour permettre à la pièce 7 de s'écarter de la pièce 6 suffisamment pour le libre passage de la tige 2 entre les mâchoires 8, 18. La largeur du passage 10 est par ailleurs légèrement supérieur à celle de la barre 4 pour autoriser une certaine adaptation angulaire entre la tige 2 et la barre 4. Ainsi, comme illustré sur la figure 1, les deux tiges 2 d'ostéosynthèse sont volontairement disposées en n'étant pas parallèles en sorte que la barre transversale 4 ne forme pas un angle droit avec les tiges 2.

Le dispositif de l'invention permet un tel montage, les pièces 6, 7 demeurant alignées sur les tiges 2, mais la barre 4 pouvant pivoter latéralement légèrement par rapport à ces pièces. Ceci évite d'avoir à cintrer la barre 4 pour avoir un croisement véritablement à angle droit avec les tiges 2.

Le déblocage et la dépose du dispositif sont tout aussi rapides et faciles puisqu'il suffit de dévisser la vis 22 et de tirer sur la barre 4 ou les pièces 6, 7 pour retirer les mâchoires 8, 18.

Comme on peut l'observer sur la figure 5, une partie substantielle (presque le tiers sur le dessin) de la périphérie de la tige 2, côté colonne vertébrale, est dégagée. Ceci, joint au fait que la forme des mâchoires 8, 18 est effilée, permet une mise en place sur les tiges 2 en n'importe quel endroit sans avoir à dégager la place nécessaire (avivement des articulaires) au voisinage immédiat des tiges. C'est ainsi que même si la tige 2 est au contact, sur sa face tournée vers le patient, avec un tissu osseux, le dispositif de l'invention sera néanmoins susceptible d'être mis en place directement et sans préparation.

Il est à noter que les pièces 6, 7 assemblées pourraient être mises en place sur la tige 2 avant insertion de la barre 4 dans le passage 10.

Il est également à noter que les possibilités d'ouverture des mâchoires 8, 18 permettent au dispositif de s'adapter à différents diamètres de tiges d'ostéosynthèse (par exemple 4, 5 ou 6 mm).

La tige 2 et la barre 4, les pièces 6, 7 et la vis 22 sont en matériau homologué pour l'implantation chirurgicale, par exemple du titane.

La barre de liaison transversale 4 a par exemple une section rectangulaire de 3 mm sur 4 mm avec des arêtes arrondies. Les formes et dimensions de la barre 4 et du passage 10 de la pièce 6 peuvent bien entendu varier , la barre 4 pouvant en particulier être carrée ou cylindrique.

Des stries peuvent être ménagés sur la partie de la barre 4 susceptible de venir en contact avec la vis 22.

Enfin, l'invention n'est évidemment pas limitée au mode de réalisation décrit et représenté mais en couvre au contraire toutes les variantes notamment en ce qui concerne les formes et dimensions des mâchoires 8, 18, le mode et les moyens d'articulation des pièces 6, 7 entre elles, ou encore le moyen exerçant une pression sur les barre 4 et pièce 7 pour assurer conjointement le blocage par rapport à la pièce 6 de la tige 2 et de la barre 4.

## Revendications

1. Dispositif de liaison rachidienne transverse, comprenant une barre transversale (4) solidarisable d'au moins une tige d'ostéosynthèse rachidienne (2) à l'aide d'un organe de liaison/blocage (5) entre ladite barre transversale et ladite tige d'ostéosynthèse, susceptible d'être rapporté sur cette dernière in situ, ledit organe de liaison/blocage étant constitué :
- d'une première pièce (6) en forme de mâchoire (8), munie d'un passage (10) pour la barre transversale (4) et d'une vis de blocage ou analogue (22) susceptible de faire saillie dans ledit passage,
- et d'une seconde pièce (7) en forme de mâchoire antagoniste (18) articulée sur la première pièce (6),
- la barre (4) en place dans le passage (10) étant susceptible, lors du serrage de ladite vis (22), de venir en appui à la fois contre la première pièce (6) et contre la seconde pièce (7) et de faire se rapprocher lesdites mâchoires (8, 18).

2. Dispositif suivant la revendication 1, **caractérisé en ce que** lesdites mâchoires (8, 18) sont de forme effilée et agencées de façon à laisser libre une partie substantielle de la périphérie de la tige d'ostéosynthèse (2).

3. Dispositif suivant la revendication 1 ou 2, **caractérisé en ce que** le passage (10) ménagé dans ladite première pièce (6) s'étend sensiblement orthogonalement à l'axe de la tige d'ostéosynthèse (2) en position entre les mâchoires (8, 18).

4. Dispositif suivant l'une des revendications 1 à 3, **caractérisé en ce que** ladite vis ou analogue (22) est disposée de l'autre côté des mâchoires (8, 18) par rapport à la barre transversale (4) et la seconde pièce (7) est articulée sur la première par une fourche (19) dans laquelle passe ladite barre transversale (4).

5. Dispositif suivant la revendication 4, **caractérisé en ce que** ladite fourche (19) se termine par deux extrémités recourbées (20) coopérant avec deux tourillons (16) prévus sur les flancs de la première pièce (6).

6. Dispositif suivant la revendication 5, **caractérisé en ce que** le débattement angulaire de la seconde pièce (7) par rapport à la première pièce (6) est limité par une goupille amovible (17).

7. Dispositif suivant l'une des revendications 1 à 6, **caractérisé en ce que** les dimensions dudit passage (10) sont sensiblement supérieures à celles de la barre transversale (4).

8. Dispositif suivant l'une des revendications 1 à 7, **caractérisé en ce que** ledit passage (10) est conformé de façon à permettre le basculement de ladite barre transversale (4) à l'intérieur du passage, autour d'un axe parallèle à l'axe d'articulation desdites pièces (6, 7), sous l'action de ladite vis ou analogue (22).

9. Dispositif suivant l'une des revendications 1 à 8, **caractérisé en ce que** ladite barre transversale (4) a une section rectangulaire ou carré.

## Patentansprüche

1. Vorrichtung für eine transversale Wirbelsäulenverbindung, die einen transversalen Stab (4) umfaßt, der an wenigstens einem Wirbelsäulen-Osteosynthesestift (2) mit Hilfe eines Verbindungs-/Blockierungsorgans (5) zwischen dem transversalen Stab und dem Osteosynthesestift, das an diesem letzteren vor Ort angefügt werden kann, befestigt werden kann, wobei das Verbindungs-/Blockierungsorgan gebildet ist aus:
- einem ersten Teil (6) in Form einer Klemmbacke (8), das mit einem Durchlaß (10) für den transversalen Stab (4) und mit einer Blockierungsschraube (22) oder dergleichen, die in den Durchlaß vorstehen kann, versehen ist,
- und einem zweiten Teil (7) in Form einer entgegenwirkenden Klemmbacke (18), die am ersten Teil (6) angelenkt ist,
- wobei sich der in den Durchlaß (10) eingesetzte Stab (4) beim Festziehen der Schraube (22) sowohl an dem ersten Teil (6) als auch an dem zweiten Teil (7) abstützen und eine Annäherung der Klemmbacken (8, 18) bewirken kann.

2. Vorrichtung nach Anspruch 1, **dadurch gekennzeichnet, daß** die Klemmbacken (8, 18) eine konisch zulaufende Form besitzen und so beschaffen sind, daß sie einen wesentlichen Abschnitt des Umfangs des Osteosynthesestifts (2) freilassen.

3. Vorrichtung nach Anspruch 1 oder 2, **dadurch gekennzeichnet, daß** sich der Durchlaß (10), der im ersten Teil (6) ausgebildet ist, im wesentlichen senkrecht zur Achse des zwischen die Klemmbacken (8, 18) eingesetzten Osteosynthesestifts (2) erstreckt.

4. Vorrichtung nach einem der Ansprüche 1 bis 3, **dadurch gekennzeichnet, daß** die Schraube (22) oder dergleichen in bezug auf den transversalen Stab (4) auf der anderen Seite der Klemmbacken (8, 18) angeordnet ist und das zweite Teil (7) am ersten Teil durch eine Gabel (19) angelenkt ist, in der der transversale Stab (4) verläuft.

5. Vorrichtung nach Anspruch 4, **dadurch gekennzeichnet, daß** die Gabel (19) in zwei zurückgebogenen Enden (20) endet, die mit zwei Drehzapfen (16) zusammenwirken, die an den Seitenflächen des ersten Teils (6) vorgesehen sind.

6. Vorrichtung nach Anspruch 5, **dadurch gekennzeichnet, daß** der Winkelausschlag des zweiten Teils (7) in bezug auf das erste Teil (6) durch einen abnehmbaren Bolzen (17) begrenzt ist.

7. Vorrichtung nach einem der Ansprüche 1 bis 6, **dadurch gekennzeichnet, daß** die Abmessungen des Durchlasses (10) wesentlich größer als jene des transversalen Stabes (4) sind.

8. Vorrichtung nach einem der Ansprüche 1 bis 7, **dadurch gekennzeichnet, daß** der Durchlaß (10) so ausgebildet ist, daß er die Schwenkung des transversalen Stabs (4) innerhalb des Durchlasses um eine zur Anlenkachse der Teile (6, 7) parallele Achse unter der Wirkung der Schraube (22) oder dergleichen zuläßt.

9. Vorrichtung nach einem der Ansprüche 1 bis 8, **dadurch gekennzeichnet, daß** der transversale Stab (4) einen rechtwinkligen oder quadratischen Querschnitt hat.

## Claims

1. A device for transverse spinal connection, comprising a transverse bar (4) secured to at least one spinal osteosynthesis rod (2) by means of a connection/blocking means (5) between said transverse bar and said osteosynthesis rod, adapted to be connected to the osteosynthesis rod in situ; the improvement wherein said connection/blocking means is constituted:
- by a first part (6) in the form of a jaw (8), provided with a passage (10) for the transverse bar (4) and with a blocking screw (22) adapted to project into said passage,
- and by a second part (7) in the form of an oppositely acting jaw (18) articulated on the first part (6),
- the bar (4) when in place in the passage (10) being adapted, upon screwing of said screw (22), to come into bearing both against the first part (6) and against the second part (7) and to cause said jaws (8, 18) to come together.

2. Device according to claim 1, wherein said jaws (8, 18) are of tapered shape and arranged so as to leave free a substantial portion of the periphery of the osteosynthesis rod (2).

3. Device according to claim 1 or to claim 2, wherein said passage (10) provided in said first part (6) extends substantially orthogonally to the axis of the osteosynthesis rod (2) in position between th jaws (8, 18).

4. Device according to anyone of claims 1-3, wherein said screw (22) is disposed on the other side of the jaws (8, 18) relative to the transverse bar (4) and the second part (7) is articulated on the first part by a fork (19) through which passes said transverse bar (4).

5. Device according to claim 4, wherein said fork (19) ends in two reversely curved ends (20) coacting with two pivots (16) provided on the sides of the first part (6).

6. Device according to claim 5, wherein the angular swinging of the second part (7) relative to the first part (6) is limited by a removable pin (17).

7. Device according to anyone of claims 1-6, wherein the dimensions of said passage (10) are substantially greater than those of the transverse bar (4).

8. Device according to anyone of claims 1-7, wherein said passage (10) is adapted to permit the swinging of said transverse bar (4) within the passage, about an axis parallel to the axis of articulation of said parts (6, 7), under the action of said screw (22).

9. Device according to anyone of claims 1-8, wherein said transverse bar (4) has a rectangular or square cross section.
